# EUROPEAN PATENT APPLICATION

(11) **EP 4 454 556 A1**
(43) Date of publication of application: **30.10.2024**
(21) Application number: 23169669.1
(22) Date of filing: 25.04.2023
(51) Int. Cl.: A61B 5/1455, A61B 5/00, A61B 7/00

(54) **METHOD AND SENSOR DEVICE FOR NON-INVASIVELY DETERMINING BLOOD OXYGEN SATURATION WITHIN TISSUE OF A HUMAN SUBJECT AND FOR MONITORING SOUND FROM INSIDE THE HUMAN SUBJECT**

(71) Applicant: CARAG AG, 6340 Baar (CH)
(72) Inventor: Larsson, Michael, 6302 Zug (CH); Bernhard, Jérôme, 8003 Zürich (CH)
(74) Representative: Grünecker Patent- und Rechtsanwälte PartG mbB

(57) **Abstract**

The invention relates to a method and a sensor device for non-invasively determining blood oxygen saturation within tissue of a human subject and for monitoring sound from inside the human subject with the sensor device, the sensor device having integrated therein an optical sensor and one or more first microphones, wherein the method comprises the steps of: transmitting, by the optical sensor, light into the tissue of the human subject, receiving, by the optical sensor, the transmitted light after it has passed through the tissue of the human subject, determining blood oxygen saturation based on the received light, and monitoring first sound from inside the human subject by using the one or more first microphones. The optical sensor integrated in the sensor device comprises one or more light sources configured for transmitting light into tissue of the human subject, and one or more light detectors configured for receiving the transmitted light after it has passed through the tissue of the human subject. The one or more first microphones integrated in the sensor device are configured for receiving first sound from inside the human subject, wherein the optical sensor and the one or more first microphones are connected to a processor, wherein the processor is configured for determining the blood oxygen saturation, and for monitoring the first sound received by the one or more first microphones.

## Description

### Field of the Invention

The present invention relates to a method and a sensor device for non-invasively determining blood oxygen saturation within tissue of a human subject and for monitoring sound from inside the human subject.

### State of the Art

In general, sensor systems using optical methods for non-invasively measuring blood oxygen saturation are known in the art. A change in blood oxygen saturation can be an indicator for a health condition. Typically, sensor systems for measuring blood oxygen saturation have multiple light sources and/or multiple light detectors in order to achieve different distances for the light to travel such that depth information of tissue can be obtained. For example, near infrared spectroscopy (NIRS) relies on the distinct absorption characteristics of oxyhemoglobin (HbO₂) and deoxyhemoglobin (Hb) in the near-infrared spectral range in order to determine the relative concentrations of HbO₂ and Hb. NIRS can be performed non-invasively by placing a spectroscopic sensor on a subject's skin and measuring the attenuation of a light signal after it has passed through the subject's tissue.

Monitoring of blood oxygen saturation within tissue of a human is of clinical importance. For example, premature babies often have immature lungs and immature intestines. In some cases, the lungs cannot oxygenate the blood adequately. The oxygen delivery to the intestines may decrease and the lining of the intestinal wall may be damaged. This allows bacteria that normally live inside the intestine to invade the intestinal wall and cause local infection and inflammation. A necrotizing enterocolitis (NEC), which is a serious intestinal disease among premature babies, may result. Although the pathogenesis of NEC is not fully resolved in the sciences, a drop in tissue oxygenation, however, can indicate an under-perfusion of the intestines, which could be an early indicator for NEC.

Further, in order to obtain information about the condition of the gastrointestinal tract, for example information about the digestion process or the peristalsis, a separate device such as a stethoscope can be used for auscultating the belly of a premature baby. For example, the degree of filling of the stomach can be obtained. This information is important, for example, for a feeding regimen, particularly when patients are artificially fed.

However, auscultation is a snap-reading method, which does not allow for a continuous monitoring of the belly. Moreover, additional information about condition of other organs such as the stomach would be of interest.

However, in order to obtain such information continuously, additional sensor(s) would be required.

### Description of the Invention

Thus, it is the object of the present invention to provide a method and a sensor device for non-invasively determining blood oxygen saturation within tissue of a human subject and for monitoring sound from inside the human subject, which allow for saving material and resources by more efficiently monitoring both blood oxygen saturation and sound with a single sensor. It is a further object of the present invention to provide a compact sensor device, which flexibly adapts to a surface of skin of a human subject.

The above problem is solved by a method for non-invasively determining blood oxygen saturation within tissue of a human subject and for monitoring sound from inside the human subject with a sensor device, wherein the sensor device having integrated therein an optical sensor and one or more first microphones, wherein the method comprises the steps of: transmitting, by the optical sensor, light into the tissue of the human subject; receiving, by the optical sensor, the transmitted light after it has passed through the tissue of the human subject; determining blood oxygen saturation based on the received light; and monitoring first sound from inside the human subject by using the one or more first microphones.

In the following the method will be described with reference to elements of the sensor device. Accordingly, some features of the sensor device will also be described.

The sensor device used for performing the method for non-invasively determining blood oxygen saturation within tissue of a human subject and for monitoring sound from inside the human subject may encapsulate the optical sensor and the one or more first microphones. The one or more first microphones may be completely encapsulated by the sensor device. The one or more first microphones may receive first sound from inside the human subject via a housing of the sensor device. Sound waves from inside the human subject may be transferred via skin to the housing contacting the skin and to the one or more first microphones contacting the housing. Alternatively, the one or more first microphones may receive the first sound by air ducts through the housing of the sensor device.

For transmitting light into the tissue of the human subject, the optical sensor may comprise one or more light sources for transmitting light and one or more light detectors for receiving the transmitted light after it has passed through the tissue of the human subject. The optical sensor may comprise a set of light sources, wherein a set of light sources may include a predetermined number of light sources. The optical sensor may comprise one or more sets of light sources. The one or more light sources and the one or more light detectors may measure the attenuation of the received light at two or more light-source-to-detector-distances. For example, the one or more light sources and the one or more light detectors may measure the attenuation of the received light at three or more distinct wavelengths in the range of 650 nm to 3 µm.

The step of monitoring first sound from inside the human subject may comprise the step of receiving, by the one or more first microphones, first sound from inside the human subject.

The one or more first microphones may comprise one or more transducers for converting sound into an electrical signal. Additionally or alternatively, the one or more first microphones may comprise at least one of optical fibers, one or more lenses, one or more analog-to-digital converters, one or more amplifiers, one or more membranes, and one or more piezo disks. A membrane and/or a piezo disk when contacting a surface of a vibrating object may convert vibrations into voltage. For example, the one or more first microphones may comprise a membrane and/or a piezo disk contacting a surface of a housing of the sensor device and receive sound from inside the human subject, since the sound waves induce vibrations that can be transferred via the housing to the membrane and/or piezo disk of the one or more first microphones.

An optical microphone consists of a membrane that is deflected by sound pressure or an acoustic signal, where the movement of the membrane is measured by the change in transmitted light intensity due to diffraction or interference.

The one or more first microphones may be, for example, at least one of an electret microphone, a Micro-Electrical-Mechanical System (MEMS) microphone, or an optical microphone.

An electret microphone may be a type of condenser microphone that has a permanently polarized capsule. The capsule, which acts as a parallel-plate capacitor, is charged via a quasi-permanent electret material applied to either the front plate (diaphragm) or stationary backplate. With a permanent charge across the plates, any change in capacitance in the electret condenser capsule creates an inversely proportional change in voltage across the plates. The capacitance of the capsule changes as the distance between the plates varies. Therefore, as the diaphragm moves back and forth, an AC voltage is created across the plates. This AC voltage is the microphone signal.

A MEMS microphone may feature pressure-sensitive diaphragms and may be built with integrated preamplifiers and/or impedance converters. The MEMS microphone may comprise a moveable membrane and a fixed backplate over a cavity in a base wafer. The MEMS diaphragm is permanently charged against a backplate. As it moves, it causes a varying capacitance between the plates. As the capacitance changes with a fixed charge in a MEMS microphone capsule, an AC voltage is produced across the plates as the microphone signal.

An optical microphone consists of a membrane that is deflected by sound pressure or an acoustic signal, where the movement of the membrane is measured by the change in transmitted light intensity due to diffraction or interference.

The optical sensor and the one or more first microphones may be connected to one or more processors. Signals from received light and/or measured attenuation of the received light and/or received first sound may be transmitted to one or more processors. The one or more processors may be integrated in the sensor device. The sensor device may be connected by cable or wirelessly to a remote digital device such as a computer or a mobile device. Signals from received light and/or measured attenuation of the received light and/or received first sound may be transmitted to one or more processors of a processing unit of a remote digital device.

The step of determining blood oxygen saturation based on the received light may comprise the step of determining the blood oxygen saturation by one or more processors. The one or more processors may be integrated in the sensor device or in a processing unit of a remote digital device.

The one or more processors may include an algorithm for determining the attenuation of the received light as a function of the wavelength and the light-source-to-detector-distance, calculating the slope of the attenuation of the received light versus the light-source-to-detector-distance as a function of the wavelength, and calculating the blood oxygen saturation within the tissue of the human subject on the basis of said slope of the attenuation of the received light and empirically determined data that account for attenuation of the received light due to light absorbers other than hemoglobin and deoxyhemoglobin in the tissue of the human subject and due to light scattering in the tissue of the subject.

Monitoring both blood oxygen saturation within the tissue of a human subject and sound from inside the human subject with a single sensor device allows for avoiding the use of additional sensors and cables. Hence, material and resources can be saved.

According to a development, the transmitting light, receiving the transmitted light and determining blood oxygen saturation may be performed continuously, and/or the monitoring first sound may be performed continuously.

The one or more light sources may transmit light into tissue of the human subject according to predetermined time intervals, the one or more light detectors may receive the transmitted light after it has passed through the tissue of the human subject accordingly, and the processor may determine the blood oxygen saturation accordingly. When the sensor device is in use, the one or more first microphones may be operated to listen to sound from inside the human subject.

According to a further development, a second sound may be monitored from outside the human subject by using one or more second microphones integrated in the sensor device.

When the sensor device is in use, the one or more second microphones may be operated to listen to sound from outside the human subject.

According to a further development, the second sound is noise from outside the human subject, and the first sound may be corrected by cancelling the noise from the first sound.

According to a further development, the sensor device may have the form of a strip.

The sensor device used for performing the method for non-invasively determining blood oxygen saturation within tissue of a human subject and for monitoring sound from inside the human subject may be shaped in the form of a flat strip. The sensor device may have the form of a straight strip. In a top view, the form of the strip may be rectangular. The length of the strip may be a multiple of a width of the strip. The sensor device may have a flat shape. The form of the sensor device may be quadratic, circular, or oval.

The sensor device may have the form of an angled strip. For example, a first portion and a second portion of the sensor device connected integrally with each other may form an angle larger than 0° and less than 180° between them.

According to a further development, the sensor device may have a first side and a second side opposite to the first side, wherein the first side is facing towards the human subject and the second side is facing away from the human subject. Each of the first and second sides may form an even surface.

According to a further development, depth information may be determined of an inside of the human subject based on the first sound monitored by using at least two of the first microphones. At least one of a location, a region, and an organ inside the human subject can be determined as a source of the first sound based on the determined depth information.

The at least two of the first microphones may be arranged spaced apart from each other within the sensor device by a predetermined distance and receive sound from inside the human subject. The depth information may be calculated based on a time delay between reception of the sound received by the at least two of the first microphones.

The invention further provides a sensor device for non-invasively determining blood oxygen saturation within tissue of a human subject and for monitoring sound from inside the human subject. The sensor device comprises: an optical sensor integrated in the sensor device, the optical sensor comprising one or more light sources configured for transmitting light into tissue of the human subject, and one or more light detectors configured for receiving the transmitted light after it has passed through the tissue of the human subject; one or more first microphones integrated in the sensor device, and configured for receiving first sound from inside the human subject; and wherein the optical sensor and the one or more first microphones are connected to a processor, wherein the processor is configured for determining the blood oxygen saturation, and for monitoring the first sound received by the one or more first microphones.

The sensor device may be configured for performing the method and the developments described above. The sensor device may be provided with one or more features that have been described above in the context with the method and developments.

The integral combination of the optical sensor and one or more first microphones within a single sensor device allows for monitoring both blood oxygen saturation within the tissue of a human subject and sound from inside the human subject, while the use of additional sensors and cables are avoided. Hence, material and resources can be saved. Beyond that, the results from the parallel measurement allow for investigating the relationship between (sounds from) peristalsis and tissue oxygenation.

According to a development, the sensor device may have a first side and a second side opposite to the first side, wherein, when in use, the first side is facing towards the human subject and the second side is facing away from the human subject.

According to a further development, each of the first and second sides may have an even surface. The sensor device may have the form of a strip.

The sensor device may be shaped in the form of a flat strip. The sensor device may have a flat shape. In a top view, the sensor device may have a rectangular form. The length of the strip may be a multiple of a width of the strip. The flat shape of the sensor device provides flexibility to the sensor device.

In a top view, the sensor device may have an angled shape. The sensor device may have a flat, angled shape. The shape of the sensor device may form an angle having a value of larger than 0° and less than 180°. Particularly, the angle may have a value of 90°to 170°.

A first portion and a second portion of the sensor device connected integrally with each other may form together the angled shape. The optical sensor may be integrated within the first portion and the one or more first microphone may be integrated within the second portion. The optical sensor and the one or more first microphones may be integrated within both the first portion and the second portion.

Alternatively the sensor device may have a flat shape and the form may be quadratic, circular, or oval.

The term "even surface" means that a top surface and a bottom surface of each element such as the optical sensor and the one or more first microphones integrated within the sensor device are integrated within a surface of the respective first and second side. In other words, the top surface and the bottom surface of each element do not project vertically beyond the sensor device in a side view. The top surface and the bottom surface of each element such as the optical sensor and the one or more first microphones integrated within the sensor device may not project horizontally beyond the sensor device in a top view. In a side view, the dimension of the sensor device may be in the range of 1 to 8 millimeters (mm).

The flat shape of the sensor device allows the sensor device for providing an increased flexibility. The sensor device may be configured to flexibly adapt to a form of skin of the human subject to which the sensor device may be attached. The sensor device may comprise a flexible plastic material. The sensor device may be attached to a diaper, a dressing or clothing. The second side of the sensor device may comprise a sticky surface. For example, the surface of the second side may be coated with an adhesive. The second side of the sensor device may comprise at least one of a hook-and-loop fastener such as a Velcro fastener and an adhesive to promote adhesion of the sensor device to a diaper, dressing or clothing.

The angled shape of the sensor device allows the sensor device to monitor a first region of the human subject, while, at the same time and by the same sensor device, a second region of the human subject may be monitored. The angled form of the sensor device allows for simultaneously monitoring different organs of the human subject at a same time.

According to a further development, the optical sensor and the one or more first microphones may be integrated in the first side of the sensor device. The first side of the sensor device may be configured for contacting a surface of the human subject.

According to a further development, the sensor device may further comprise at least one of a motion sensor, a temperature sensor, an Analog-to-Digital, A/D-, converter, a wireless module, and a power source. The temperature sensor may be configured for measuring a temperature value of a region of skin of the human subject, the region of skin being irradiated with light transmitted by the one or more light sources, and the sensor device may be configured to switch off the one or more light sources when the temperature value of the irradiated region of skin exceeds a preset threshold value.

The motion sensor, temperature sensor, Analog-to-Digital, A/D-, converter, wireless module, and/or the power source may be elements that are integrated in the sensor device and do not project beyond an outer surface of the sensor device.

The power source may be a battery. The temperature sensor may be arranged in close proximity to the one or more light sources. The temperature sensor may measure a temperature value of the region of skin of the human subject during operation of the one or more light sources. The processor may receive a signal from the temperature sensor. In response to the received signal, the processor may control operation of the one or more light sources. The temperature sensor may measure a temperature value of the region of skin of the human subject while the one or more light sources are not operated. The processor may be configured for calculating a core body temperature of the human subject based on the measured temperature value received from the temperature sensor while the one or more light sources are not operated.

Alternatively, a first temperature sensor may measure a first temperature value of a region of skin of the human subject, the region of skin being irradiated with light transmitted by the one or more light sources, and a second temperature sensor may measure a second temperature value of a region of skin of the human subject, the region of skin being not irradiated with light transmitted by the one or more light sources. The second temperature value may be used to calculate a core body temperature of the human subject.

The one or more light sources may cause an increase of the temperature of skin being irradiated by the one or more light sources. By switching off the one or more light sources, arising pain due to the increase of the skin temperature and damage of skin tissue of the human subject may be prevented.

According to a further development, the sensor device may comprise one or more second microphones integrated in the sensor device, which may be configured for monitoring second sound from outside the human subject. The one or more second microphones may be integrated in the second side of the sensor device, and may not project beyond an outer surface of the sensor device. Alternatively or additionally, the one or more second microphones may be integrated in a frame of the sensor device, which may laterally surround the first and second sides. The first and the second sides of the sensor device may define a width and a length of the sensor device. The frame may be provided with rounded edges to an outside of the sensor device.

The one or more second microphones may listen to sound from outside the human subject for monitoring second sound. The second sound may be noise from outside the human subject.

The processor may be configured for correcting the first sound by cancelling the second sound from the first sound.

The sensor device may be used for non-invasively determining blood oxygen saturation within tissue of a human subject and for monitoring sound from inside the human subject, wherein the one or more light sources are used for transmitting light into tissue of the human subject, and the one or more light detectors are used for receiving the transmitted light after it has passed through the tissue of the human subject. The one or more first microphones are used for receiving first sound from inside the human subject, and wherein the one or more first microphones are connected to a processor, wherein the processor is used for determining the blood oxygen saturation, and for monitoring the first sound received by the one or more first microphones.

According to a development, the first side of the sensor device may contact the human subject. The one or more second microphones may be used for monitoring second sound from outside the human subject.

Further features and advantages of the present invention will be described in the following with reference to the figures, which illustrate only examples of embodiments of the present invention. The illustrated and described features may be suitably combined with each other.

### Drawings

- Fig. 1a: is an exploded view of elements of a sensor device;
- Fig. 1b: is a perspective view of a sensor device;
- Fig. 1c: is a sectional perspective view of a sensor device;
- Fig. 2a: is an exploded view of elements of a sensor device;
- Fig. 2b: is a perspective view of a sensor device;
- Fig. 2c: is a sectional perspective view of a sensor device;
- Fig. 3: is a schematic plan view of a sensor device;
- Fig. 4a: is a schematic plan view of a sensor device;
- Fig. 4b: is a schematic plan view of a sensor device;
- Fig. 5: is a schematic plan view of a sensor device;
- Fig. 6: is a schematic plan view of a sensor device;
- Fig. 7: is a schematic side view of a sensor device;
- Fig. 8a: is a schematic plan view of a sensor device;
- Fig. 8b: is a schematic plan view of a sensor device;
- Fig. 8c: is a schematic plan view of a sensor device;
- Fig. 9: is a schematic plan view of a sensor device;
- Fig. 10: is a schematic plan view of a sensor device;
- Fig. 11: is a schematic plan view of a sensor device;
- Fig. 12a: is a schematic plan view of a sensor device;
- Fig. 12b: is a schematic plan view of a sensor device;
- Fig. 13: is a schematic plan view of a sensor device;
- Fig. 14: is a schematic plan view of a sensor device;
- Fig. 15: is a schematic plan view of a sensor device;
- Fig. 16: is a schematic plan view of a sensor device;
- Fig. 17: is a schematic plan view of a sensor device;
- Fig. 18: is a schematic side view of a sensor device;
- Fig. 19: is a schematic plan view of a sensor device;
- Fig. 20: is a schematic plan view of a sensor device;
- Fig. 21: is a schematic plan view of a sensor device;
- Fig. 22: is a schematic plan view of a sensor device;
- Fig. 23: is a schematic plan view of a sensor device;
- Fig. 24: is a schematic diagram of a sensor device and a processing unit.

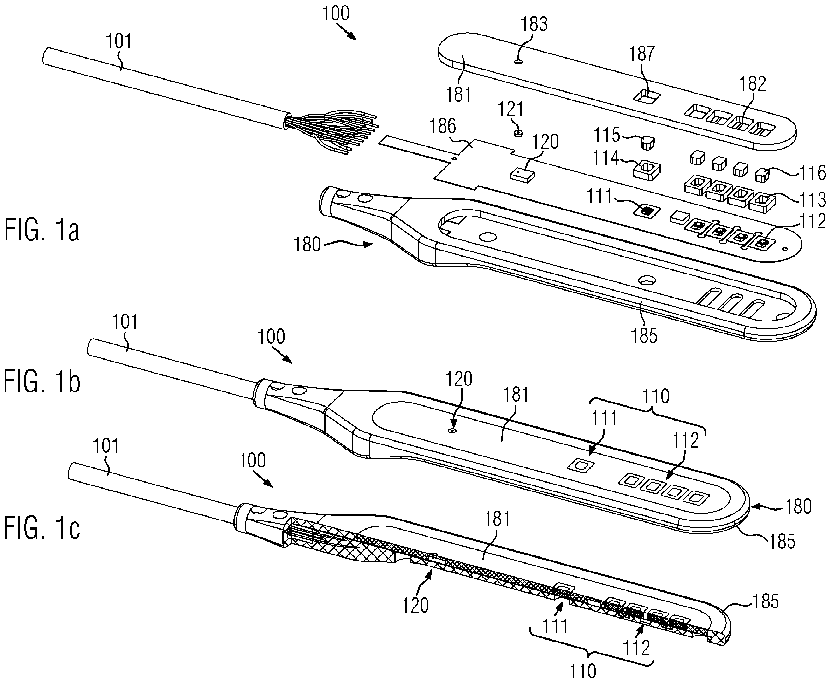

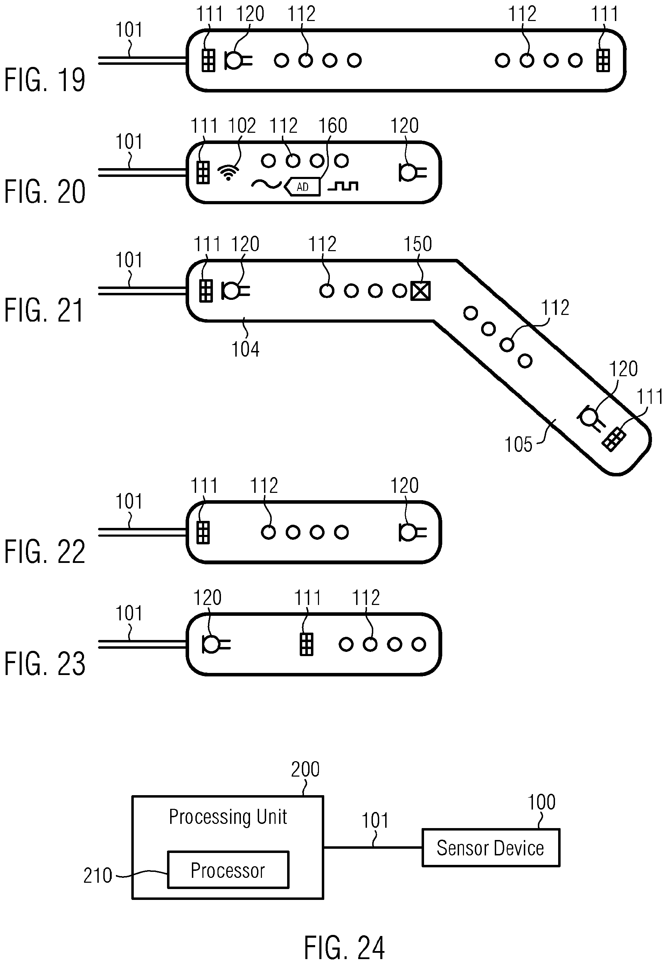

In Figs. 1a to 1c, a sensor device 100 is shown. Fig. 1a is an exploded view of components of the sensor device 100. The sensor device 100 comprises a housing 180, which accommodates a plate 186, a first microphone 120, light sources 111, light detectors 112, a mount 114, an optical window 115, mounts 113, optical windows 116, and a cable 101. The first microphone 120, light sources 111, and light detectors 112 are disposed on the plate 186. The plate 186 may comprise a printed circuit board (PCB). The housing 180 comprises a cover 181 and a frame 185. The cover 181 comprises a through hole 183 for a mount 121 for the first microphone 120, a recess 187 for accommodating the mount 114, which integrates the optical window 115 above the light sources 111, and recesses 182 for accommodating the mounts 113, which integrate the optical windows 116 above the light detectors 112. The first microphone 120 is aligned with the through hole 183 by the mount 121. An optical sensor comprises the light sources 111, the light detectors 112, the mount 114 for the optical window 115 for the light sources 111, the optical window 115, the mounts 113 for the optical windows 116 for the light detectors 112, and the optical windows 116. The sensor device 100 may be connected via a cable 101 to a processing unit 200 as shown in Fig. 24. Fig. 1b is a perspective view of the sensor device 100 shown in Fig. 1a. The sensor device 100 has a flat shape and an even surface. The housing 180 encapsulates the first microphone 120 and the optical sensor 110 comprising the light sources 111 and the light detectors 112. The frame 185 encloses the plate 186 and the cover 181 at their side edges. Fig. 1c is a sectional perspective view of the sensor device 100 shown in Figs. 1a and 1b.

In Figs. 1a to 1c, first microphone 120 can receive sound from inside the human subject via an air duct provided by through hole 183. The sensor device 100 comprises a cable 101 in Figs. 1a to 1c, however, alternatively, the sensor device 100 may be wirelessly connected to a processing unit.

The sensor device 100 may have the form of a strip having rounded edges. The sensor device 100 may comprise additional elements such as at least one of a processor, further light sources, further light detectors, microphone(s), temperature sensor(s), motion sensor(s), a wireless module, a power source, and A/D converter(s).

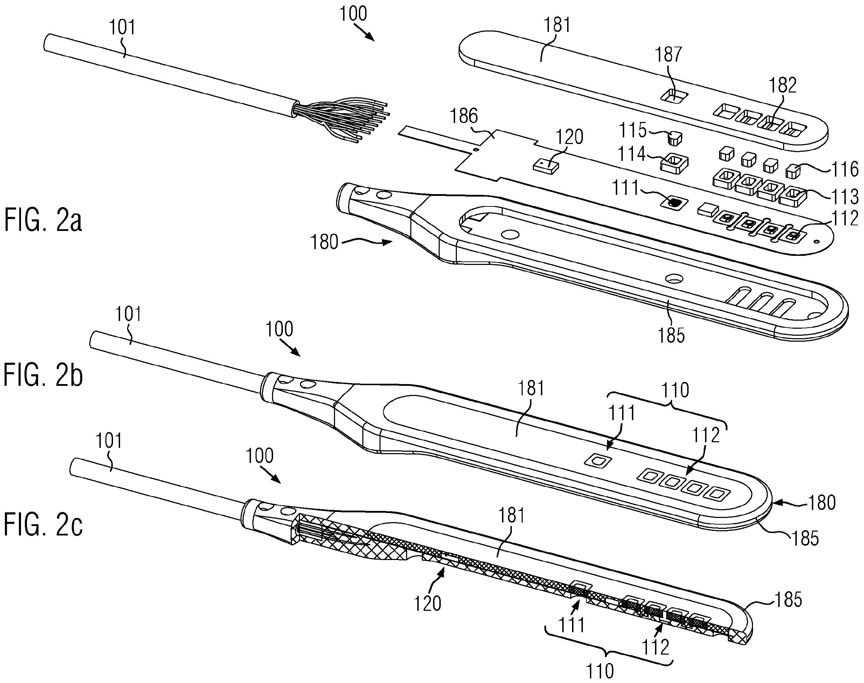

In Figs. 2a to 2c, a sensor device 100 is shown. The sensor device 100 of Figs. 2a to 2c comprises the same elements as the sensor device 100 of Figs. 1a to 1c except for the through hole 183 and the mount 121 for the first microphone 120. Fig. 2a is an exploded view of elements of the sensor device 100. Fig. 2b is a perspective view of the sensor device 100 shown in Fig. 2a, and Fig. 2c is a sectional perspective view of the sensor device 100 shown in Figs. 2a and 2b. In Figs. 2a to 2c, the cover 181 does not comprise through hole 183 for the first microphone 120. Sound from inside the human subject can be transferred via the cover 181 to the first microphone 120. In Figs. 2a to 2c, the sensor device 100 comprises a cable 101 and may be connected to a processing unit 200 as shown in Fig. 24. However, the sensor device 100 may be wirelessly connected to a processing unit.

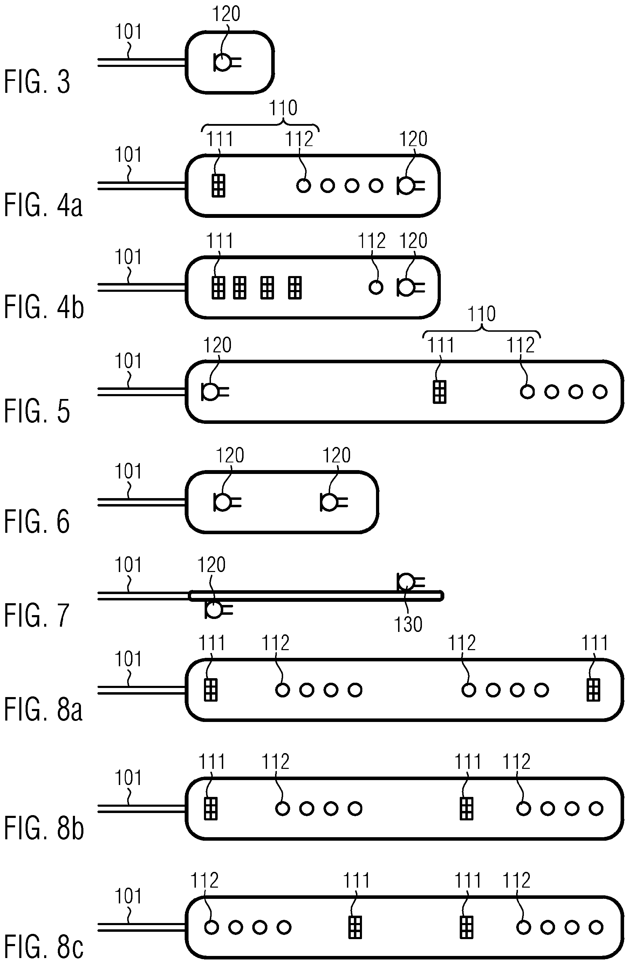

Figs. 3 to 23 show several examples of the sensor device 100. Although Figs. 3 to 23 depict specific elements of the sensor device 100, these specific elements can be combined with one or more other elements within the sensor device 100. The elements may comprise at least one of a processor, one or more light sources, one or more sets of light sources, one or more light detectors, one or more microphones, one or more temperature sensors, one or more motion sensors, a wireless module, a power source, and an A/D converter. In Figs. 3 to 23, the sensor device 100 is shown having a number of a certain type of elements. However, the sensor device 100 is not limited thereto. Fig. 3 is a schematic plan view of the sensor device 100. In Fig. 3, first microphone 120 and cable 101 of the sensor device 100 are depicted. However, sensor device 100 may comprise additional elements. Fig. 4a is a schematic plan view of a sensor device 100 comprising first microphone 120 and optical sensor 110 having a set of light sources 111 and light detectors 112. In Fig. 4a, a set of light sources 111 and four light detectors 112 are shown. However, the number of light sources and the number of light detectors is not limited thereto. For example, the number of sets of light sources may be four and the number of light detectors may be one, as shown in Fig. 4b. In Fig. 4b, the sensor device 100 also comprises first microphone 120. Fig. 5 is a schematic plan view of a sensor device 100 and shows another example of the sensor device 100. The sensor device 100 comprises a first microphone 120, a set of light sources 111 and light detectors 112. In Fig. 5, first microphone 120 and the optical sensor 110 comprising the set of light sources 111 and the light detectors 112 are spaced apart by a predetermined distance. Fig. 6 is a schematic plan view of a sensor device 100. In Fig. 6, two of first microphones 120 are shown. The optical sensor is not shown in Fig. 6 for a better overview of the described components. By providing two of the first microphones 120, depth information may be determined of an inside of the human subject. Sound may be monitored by the first microphones 120. As shown in Fig. 6, the two of the first microphones 120 may be arranged spaced apart from each other within the sensor device 100 and receive sound from inside the human subject. The depth information may be calculated based on a time delay between receptions of the sound received by the two of the first microphones 120. At least one of a location, a region, and an organ inside the human subject may be determined as a source of the sound from inside the human subject based on the determined depth information. The sensor device 100 shown in Fig. 6 may comprise additional elements.

Fig. 7 is a schematic side view of a sensor device 100. The sensor device 100 comprises a first microphone 120 for listening to sound from inside the human subject, and a second microphone 130 for listening to sound from outside the human subject. The sound from outside the human subject may be noise. The sound from inside the human subject received by the first microphone 120 may be corrected by cancelling the noise received by the second microphone 130 from the sound from inside the human subject.

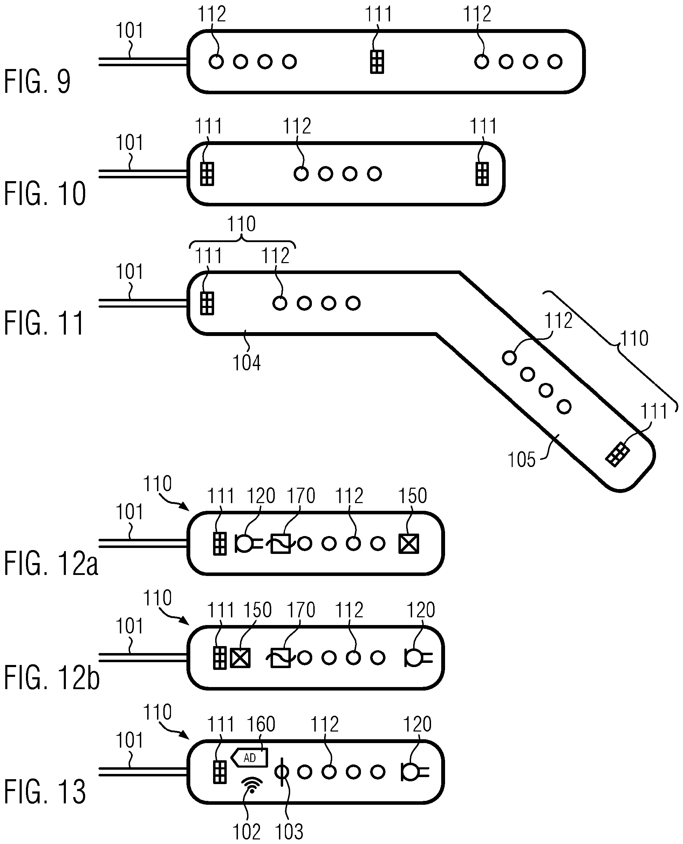

Figs. 8a to 8c are schematic plan views of a sensor device 100 illustrating examples of different possible arrangements of light sources 111 and light detectors 112. Figs. 9 and 10 are schematic plan views of a sensor device 100 illustrating different arrangements and numbers of sets of light sources 111 and light detectors 112.

Figs. 4 to 10 show a sensor device 100 having a cable 101. However, the sensor device 100 may be provided without cable 101. For example, the sensor device 100 may additionally comprise a wireless module and/or a power source.

Fig. 11 is a schematic plan view of a sensor device 100 and schematically shows another example. The sensor device 100 has an angled shape. The sensor device 100 is shaped in an angled form. The sensor device 100 comprises a first portion 104 and a second portion 105 forming together an angle. The angle formed between the first portion 104 and the second portion 105 may have a value, for example, in a range of 45° to 170°.

The angled form of the sensor device 100 may be advantageous in order to monitor several organs of the human subject with the sensor device 100 at the same time. For example, when the human subject is a baby, the sensor device 100 may be attached to the skin of the baby. The first portion 104 of the sensor device 100 may comprise an acoustical sensor including one or more first microphones 120 and the second portion 105 of the sensor device 100 may comprise the optical sensor 110 having one or more light sources 111 and one or more detectors 112. The second portion 105 of the sensor device 100 may be fixed by a diaper worn by the baby and the optical sensor 110 may monitor blood oxygen saturation within the tissue of the intestine of the baby, while sound from inside the stomach of the baby may be monitored by the one or more first microphones 120 of the acoustical sensor integrated in the first portion 104 of the sensor device 100.

Figs. 12a and 12b are schematic plan views of a sensor device 100. The sensor device 100 comprises an optical sensor 110 having a set of light sources 111 and light detectors 112, a first microphone 120, a motion sensor 170, and a temperature sensor 150. The arrangement of the elements of the sensor device 100 shown in Figs. 12a and 12b, may vary according to requirements served by the sensor device 100.

Fig. 13 is a schematic plan view of a sensor device 100, which comprises an optical sensor 110 including a set of light sources 111 and light detectors 112, a wireless module 102, a first microphone 120, an A/D converter 160, and a power source 103. The power source 103 may be a battery.

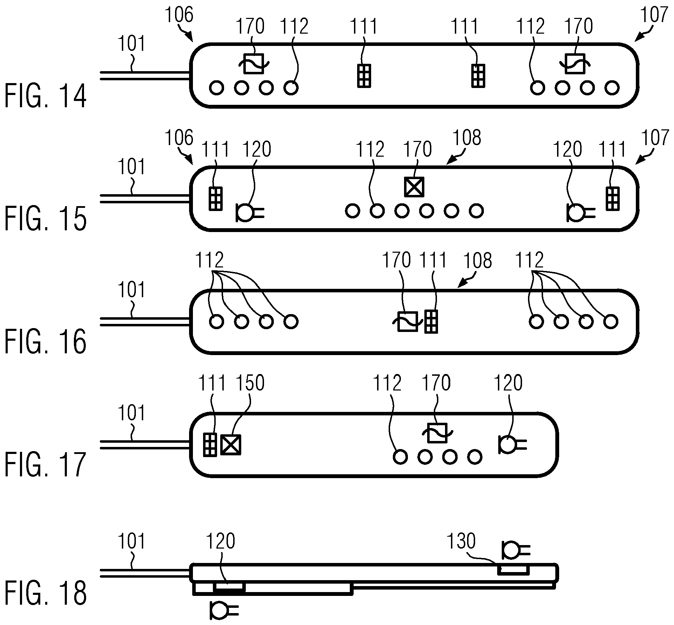

Figs. 14 to 17 are schematic plan views of a sensor device 100. In Figs. 14 to 17, the sensor device 100 may comprise differing elements in diverse arrangements. In Fig. 14, a first motion sensor 170 and first light detectors 112 are integrated in a first end region 106 of the sensor device 100. A second motion sensor 170 and second light detectors 112 are integrated in a second end region 107 of the sensor device 100. A first set of light sources 111 and a second set of light sources 111 are integrated in a middle region of the sensor device 100. In Fig. 15, a temperature sensor 150 and light detectors 112 are arranged in a middle region 108 of the sensor device 100, wherein the temperature sensor 150 is disposed to be in close proximity to the light detectors 112. In Fig. 16, a set of light sources 111 and a motion sensor 170 are arranged in the middle region 108 of the sensor device 100. Additionally, a temperature sensor 150 may be integrated in the middle region 108. Based on the two microphones 120 depth information may be obtained. The sensor device 100 shown in Fig. 17, comprises a set of light sources 111, light detectors 112, a first microphone 120, a temperature sensor 150, and a motion sensor 170. The temperature sensor 150 is disposed in close proximity to the set of light sources 111. The temperature sensor may measure a temperature value of a region of skin, which is irradiated with light transmitted by the set of light sources 111.

Fig. 18 is a schematic side view of a sensor device 100. In Fig. 18, the sensor device 100 comprises a first microphone 120 for receiving sound from inside the human subject and a second microphone 130 for receiving sound from outside the human subject. As shown in Fig. 18, the first microphone 120 and the second microphone 130 are integrated within the sensor device 100 and do not extend beyond the surface of the sensor device 100, which has a flat shape. In Fig. 18, the first microphone 120 and the second microphone 130 are depicted, however, the sensor device 100 may comprise additional elements.

Figs. 19 to 23 are schematic plan views of a sensor device 100. The sensor device 100 shown in Fig. 19, comprises a set of light sources 111, a first microphone 120, and light detectors 112 at one end portion of the sensor device 100, and additional detectors 112 and an additional set of light sources 111 at the other end portion of the sensor device 100. In Fig. 20, the sensor device 100 comprises a set of light sources 111, a wireless module 102, light detectors 112, an A/D converter 160 and a first microphone 120. In Fig. 21, the sensor device 100 has an angled shape. A first portion 104 and a second portion 105 of the sensor device 100 form an angle between them. Each of the first portion 104 and the second portion 105 comprises a light source 111, a first microphone 120 and light detectors 112. A temperature sensor 150 is arranged in a middle portion of the sensor device 100. The middle portion of the sensor device 100 is a portion, which connects the first portion 104 and the second portion 105. In the sensor device 100 shown in Fig. 22, a set of light sources 111 is arranged at an end portion of the sensor device 100 and a first microphone 120 is arranged at the other end portion of the sensor device 100, wherein the detectors 112 are disposed between the set of light sources 111 and the first microphone 120. Fig. 23 illustrates another example for an arrangement of the first microphone 120, the set of light sources 111 and the detectors 112.

Fig. 24 is a schematic diagram of a sensor device 100, which is connected via a cable 101 to a processing unit 200. Alternatively, the sensor device 100 may be wirelessly connected to processing unit 200. The processing unit 200 comprises a processor 210. The sensor device 100 may comprise elements as described above, such as an optical sensor 110, which may comprise one or more light sources 111 and one or more light detectors 112, and an acoustic sensor, which may comprise one or more first microphones 120. The acoustic sensor may additionally comprise one or more second microphones 130. The sensor device 100 may comprise housing 180. The sensor device 100 may comprise additional elements. The optical sensor 110 and the acoustic sensor as well as additional elements may be integrated within the housing 180 of the sensor device 100. In Fig. 24, the sensor device 100 transmits measurement results obtained by the optical sensor 110 and the acoustic sensor via cable 101 to processing unit 200. The processing unit 200 may process the measurement results received from the sensor device 100 and determine blood oxygen saturation within tissue of a human subject, monitor sound from inside a human subject, and/or determine at least one of a location, a region, and an organ inside the human subject as a source of sound by determining depth information. Depth information may be obtained by using at least two of the first microphones 120. The processing unit 200 may correct sound from inside the human subject received from the sensor device 100 by cancelling sound from outside the human subject received from the sensor device 100 from the sound from inside the human subject.

Each sensor device 100 shown in Figs. 1 to 24 may comprise additional elements, which include at least one of a processor, one or more optical sensors, one or more first microphones, one or more second microphones, one or more light sources, one or more light detectors, one or more temperature sensors, one or more motion sensors, one or more A/D converters, a wireless module, and a power source. Each sensor device 100 shown in the Figures, which comprises a cable 101 may, alternatively, be provided with a power source 103 and a wireless module 102 instead of cable 101.

## Claims

1. Method for non-invasively determining blood oxygen saturation within tissue of a human subject and for monitoring sound from inside the human subject with a sensor device, the sensor device having integrated therein an optical sensor and one or more first microphones, wherein the method comprises the steps of:
transmitting, by the optical sensor, light into the tissue of the human subject;
receiving, by the optical sensor, the transmitted light after it has passed through the tissue of the human subject;
determining blood oxygen saturation based on the received light; and
monitoring first sound from inside the human subject by using the one or more first microphones.

2. The method of claim 1, wherein the steps of transmitting light, receiving the transmitted light and determining blood oxygen saturation are performed continuously; and/or
the step of monitoring first sound is performed continuously.

3. The method of claim 1 or 2, further comprising the step of monitoring second sound from outside the human subject by using one or more second microphones integrated in the sensor device.

4. The method of claim 3, wherein the second sound is noise from outside the human subject, and the method further comprising the step of correcting the first sound by cancelling the noise from the first sound.

5. The method of anyone of the preceding claims, wherein the sensor device has the form of a strip.

6. The method of anyone of the preceding claims, wherein the sensor device has a first side and a second side opposite to the first side, wherein the first side is facing towards the human subject and the second side is facing away from the human subject;
preferably, wherein each of the first and second sides form an even surface.

7. The method of anyone of the preceding claims, further comprising the step of determining depth information of an inside of the human subject based on the first sound monitored by using at least two of the first microphones;
preferably, wherein the step of determining depth information comprises determining at least one of a location, a region, and an organ inside the human subject as a source of the first sound based on the determined depth information.

8. A sensor device for non-invasively determining blood oxygen saturation within tissue of a human subject and for monitoring sound from inside the human subject, the sensor device comprising:
an optical sensor integrated in the sensor device, the optical sensor comprising one or more light sources configured for transmitting light into tissue of the human subject, and one or more light detectors configured for receiving the transmitted light after it has passed through the tissue of the human subject;
one or more first microphones integrated in the sensor device, and configured for receiving first sound from inside the human subject; and
wherein the optical sensor and the one or more first microphones are connected to a processor, wherein the processor is configured for determining the blood oxygen saturation, and for monitoring the first sound received by the one or more first microphones.

9. The sensor device of claim 8, wherein the sensor device has a first side and a second side opposite to the first side, wherein, when in use, the first side is facing towards the human subject and the second side is facing away from the human subject.

10. The sensor device of claim 8 or 9, wherein each of the first and second sides has an even surface; and/or
wherein the sensor device has the form of a strip.

11. The sensor device of one of claims 8 to 10, wherein the optical sensor and the one or more first microphones are integrated in the first side of the sensor device; and/or wherein the first side of the sensor device is configured for contacting a surface of the human subject.

12. The sensor device of one of claims 8 to 11, further comprising at least one of a motion sensor, a temperature sensor, an Analog-to-Digital, A/D-, converter, a wireless module, and a power source,
preferably, wherein the temperature sensor is configured for measuring a temperature value of a region of skin of the human subject, the region of skin being irradiated with light transmitted by the one or more light sources, and the sensor device being configured to switch off the one or more light sources when the temperature value of the irradiated region of skin exceeds a preset threshold value.

13. The sensor device of one of claims 9 to 12, further comprising one or more second microphones integrated in the sensor device, and configured for monitoring second sound from outside the human subject;
preferably, wherein the one or more second microphones are integrated in the second side of the sensor device.

14. Use of the sensor device of one of claims 8 to 13 for non-invasively determining blood oxygen saturation within tissue of a human subject and for monitoring sound from inside the human subject, wherein the one or more light sources are used for transmitting light into tissue of the human subject, and the one or more light detectors are used for receiving the transmitted light after it has passed through the tissue of the human subject;
the one or more first microphones are used for receiving first sound from inside the human subject; and
wherein the one or more first microphones are connected to a processor, wherein the processor is used for determining the blood oxygen saturation, and for monitoring the first sound received by the one or more first microphones.

15. The use of the sensor device of claim 14, wherein the first side of the sensor device is contacting the human subject; and/or
the one or more second microphones are used for monitoring second sound from outside the human subject.
